(19) Europäisches Patentamt
European Patent Office
Office européen des brevets

(11) Publication number: 0 338 591 A2

(12) # EUROPEAN PATENT APPLICATION

(21) Application number: 89107273.8

(22) Date of filing: 21.04.89

(51) Int. Cl.4: C12Q 1/68 , C12P 19/34 , C07H 1/08

(30) Priority: 21.04.88 US 184467

(43) Date of publication of application: 25.10.89 Bulletin 89/43

(84) Designated Contracting States: AT BE CH DE ES FR GB GR IT LI LU NL SE

(71) Applicant: MICROPROBE CORPORATION
1725-220th Street S.E. Suite 104
Bothell, WA 98021(US)

(72) Inventor: Vermeulen, Nicolaas M.J.
19334 196th Avenue, N.E.
Woodinville Washington 98072(US)
Inventor: Schwartz, Dennis E.
20621 N.E. 37th Way
Redmond Washington 98053(US)

(74) Representative: Dipl.-Phys.Dr. Manitz
Dipl.-Ing., Dipl.-W.-Ing. Finsterwald Dipl.-Ing.
Grämkow Dipl.-Chem.Dr. Heyn Dipl.-Phys.
Rotermund
Morgan, B.Sc.(Phys.) Robert-Koch-Strasse 1
D-8000 München 22(DE)

(54) **Nucleic acid extraction method.**

(57) Nucleic acids are extracted from biological samples by pretreating the samples to release the nucleic acids and contaminating materials into an aqueous phase. Optionally, the samples may be extracted with an anionic detergent in an organic solvent prior to release of the nucleic acids. The aqueous phase is then combined with an organic phase in the presence of a cationic detergent capable of forming water insoluble complexes with the nucleic acids. Usually, a chelant will also be combined with the aqueous phase. The complexes are preferentially partitioned into the organic phase and are thus separated from the contaminating materials which remain in the aqueous phase. The nucleic acids may then be transferred back to a second aqueous phase by combining the organic phase with water in the presence of an inorganic salt selected to displace the detergent cation. After separation of the organic phase, the second aqueous phase containing the nucleic acids may be used for conventional hybridization testing.

EP 0 338 591 A2

# NUCLEIC ACID EXTRACTION METHOD

## BACKGROUND OF THE INVENTION

### 1. Field of the Invention

The present invention relates generally to microbial identification methods, and more particularly to methods for extracting nucleic acids from complex biological samples prior to performing specific hybridization assays.

The detection of pathogenic microorganisms in biological specimens using nucleic acid hybridization assays is becoming increasingly widespread. Such assays are relatively simple in theory, requiring only that nucleic acids be extracted from the specimen and thereafter combined with detectable nucleic acid probes characteristic of the microorganism. By detecting specific binding between the probes and the extracted nucleic acids, presence of the microorganism in the specimen can be established. In practice, of course, numerous difficulties arise in the performance of such assays.

The present invention is concerned with methods for extracting the nucleic acids from the biological specimens prior to performance of the hybridization assays. Heretofore, nucleic acid extraction has usually relied on phenol extraction combined with centrifugal separation and ethanol precipitation. Although generally workable, such multiple step extraction methods are cumbersome and time-consuming. Moreover, phenol is toxic and such extraction processes can be hazardous to personnel. Finally, phenol extraction can leave relatively large amounts of undesirable substances in the final assay medium, particularly when the nucleic acids are extracted from complex biological specimens, such as feces or sputum.

For the above reasons, it would be desirable to provide methods for extracting nucleic acids from biological samples which are rapid, which do not employ hazardous reagents, and which provide a clean separation of the nucleic acids with minimal carry-over of contaminating substances from the biological sample to the assay medium.

### 2. Description of the Background Art

Langridge et al. (1980) Anal. Biochem. 103:264-271 describes a preparative technique for extracting nucleic acids from agarose electrophoresis gels. Quaternary ammonium salts of the nucleic acids are partitioned into 1-butanol. The nucleic acids are then converted back into their sodium salts and recovered in an aqueous phase. Hurst and Sheng (1976) Biochim. Biophys. Acta 444:75-84 describes the partitioning of complexes of polyanionic glycosaminoglycans and quaternary ammonium cations between aqueous and alcoholic phases based on changes in salt concentration. Luisi (1985) Angew. Chem. Int. Ed. Engl. 24:439-450 describes in Fig. 15, an apparatus having a pair of vertical columns joined by a horizontal bridge for transporting proteins between two aqueous phases.

## SUMMARY OF THE INVENTION

Extraction of nucleic acids from biological samples prior to nucleic acid hybridization testing comprises pretreating the sample to release nucleic acids into a first aqueous phase. By adding a cationic detergent to the first aqueous phase, nucleic acid complexes are formed which are preferentially soluble in an organic phase. The organic phase may be combined with the first aqueous phase simultaneously with or subsequent to the addition of the cationic detergent. In the latter case, the nucleic acid complexes will precipitate in the presence of low salt concentrations prior to combination with the organic phase, providing an additional separation step. Suitable cationic detergents include quaternary ammonium salts, quaternary phosphonium salts, amine oxides, pyridinium-derivatives, and the like. Optionally, a chelant may be added to the first aqueous phase to sequester divalent cations which might otherwise compete with the cationic detergent for binding to the nucleic acids. Additionally, the sample may be pre-extracted with an anionic or non-ionic detergent in an organic solvent prior to extraction with the cationic detergent.

The organic phase is separated from the first aqueous phase and mixed with an inorganic salt in a second aqueous phase. The nucleic acid complexes are dissociated from the cationic detergent by

displacement with the inorganic salt, allowing the nucleic acids to partition into the second aqueous phase. The second aqueous phase can then be separated from the organic phase and serve as the assay medium for conventional nucleic acid hybridization.

The method of the present invention is advantageous in that it provides improved separation of the nucleic acids from the biological samples, particularly in the case of complex biological samples such as feces and sputum where large amounts of potentially contaminating materials are initially present. Additionally, the method is non-hazardous to those involved in its performance as it does not require the use of toxic or otherwise dangerous reagents. Finally, the extraction protocol is simple and rapid. It may be performed with as few as two mixing and two separation steps, without the need to precipitate the nucleic acids and separate by centrifugation (although centrifugation may be performed in some cases).

In the preferred embodiment, biological samples are suspended in a suitable buffer and pretreated with a detergent and enzyme (such as a lysozyme, cellulase and/or protease) to disrupt the outer membrane or coat of the pathogen and release nucleic acids, leaving a substantial residue of potentially contaminating substances (which are usually present in excess mass over the nucleic acids). The pretreated sample is optionally mixed with an anionic detergent, such as SDS, in an organic phase, typically an alcohol or alcohol/alkane mixture, in order to remove proteins and other cationic contaminants. The aqueous phase is then separated and combined with an organic phase containing the cationic detergent which combines with the nucleic acids to form complexes which are substantially insoluble in water but soluble in the organic phase. A chelant, such as EDTA, will usually be present in order to inhibit binding between the nucleic acids and any endogenous divalent cations. The nucleic acid-detergent complex preferentially passes to the organic phase, and the organic phase is separated from the aqueous phase leaving the non-nucleic acid residue in the first aqueous phase. The nucleic acids may then be transferred back to a second aqueous phase by combination with the second aqueous phase in the presence of an inorganic salt which displaces the charged detergent and thus causes the detergent-nucleic acid complex to dissociate. A nucleic acid hybridization assay may then be performed on the second aqueous phase in a conventional manner. The assay normally provides a much lower background than if alternative nucleic acid extraction methods had been employed.

## BRIEF DESCRIPTION OF THE DRAWINGS

Fig. 1 illustrates a device and method for performing nucleic acid extractions according to the present invention without centrifugation.

Fig. 2 illustrates a method for performing nucleic acid extractions according to the present invention with continuous separation.

## DESCRIPTION OF THE SPECIFIC EMBODIMENTS

Methods are provided for extracting nucleic acids from complex biological samples and performing specific nucleic acid hybridization assays. The methods rely on combining a sample pretreated to release the nucleic acids with a cationic detergent, usually in the presence of a chelant, in a first aqueous phase. The resulting nucleic acid complexes are substantially insoluble in low salt aqueous solutions and will partition into an admixed organic phase, leaving substantially all of the non-nucleic acid residue of the sample material in the first aqueous phase. The nucleic acids may then be recovered from the organic phase by combination with a second aqueous phase in the presence of an inorganic salt which effects a sharp transition in the solubility of the complex so that the nucleic acids enter the second aqueous phase. Conventional hybridization assays may then be performed on the nucleic acids in the second aqueous phase.

The biological samples of interest include virtually any type of specimen which may contain microorganisms of interest. Exemplary biological samples include fecal samples, blood, plasma, serum, urine, sputum, semen, tissue samples, e.g., biopsies, and the like. Although primarily useful in detecting viral and bacterial microorganisms present in a sample, the method of the present invention may also be used for detecting endogenous nucleic acids in a host of interest, i.e., from a cellular sample of the host. The method of the present invention will not differ significantly depending on whether endogenous or exogenous (microorganism) nucleic acids are being detected. The extraction method of the present invention will result

in the release of most nucleic acids present in the sample regardless of source.

Pathogenic viruses and microorganisms which may be detected in fecal and gastric samples, or as contaminants of foods, beverages or water, or the like, and include viruses, such as Adenovirus, Rotavirus, and the like; bacteria, such as *Salmonella* ssp. *(S. typhimurium, S. typhi, S. paratyphi A*, etc.), *Shigella* spp. *(S. dysenteriae S. flexneri, S. boydii, S. sonnei*, etc.), *Campylobacter* spp. *(C. jejuni, C. coli C. pylori), Clostridium difficile, Escherichia coli, Yersinia enterolitica*, and the like; fungi, such as *Candida albicans*, and the like; and protozoans, such as *Giardia lamblia, Entamoeba histolytica,Microsporidium* spp., and the like.

Pathogenic viruses and microorganisms infecting the lung, bronchial and upper respiratory areas which may be detected in saliva, sputum or respiratory lavage samples include viruses, such as Adenovirus, Respiratory Syncytial Virus, Human Papillovavirus, Human Immunodeficiency Virus, Human T-Cell Lymphotrophic Viruses, Cytomegalovirus, Hepatitis A and B Virus, Epstein-Barr Virus, and the like; bacteria, such as *Streptococcus pyrogenes* (Group A beta-hemolytic streptococci), *Streptococcus pneumoniae, Haemophilus influenzae, Staphylococcus aureus, Klebsiella pneumoniae, Pseudomonas aeruginosa, Neisseria meningitidis, Neisseria gonorrhoea, Chlamydia trachomatis Escherichia coli, Mycobacterium tuberculosis, Spirochaetales*, such as *Treponema* spp., and the like; fungi, such as *Candida albicans, Histoplasma capsulatum, Coccidiodes imitis*, and the like; and, protozoans, such as *Pneumocystis carinii*, and the like.

Pathogenic viruses and microorganisms, which may be detected in blood (cells, serum and/or plasma), and include: viruses, such as Human Immunodeficiency Virus, Hepatitis A and B Virus, Human T-Cell Lymphotrophic Viruses, and the like; bacteria, such as *Escherichia coli, Staphylococcus aureus, Klebsiella pneumoniae, Streptococcus peumoniae, Pseudomonas aeruginos*a, Spirochaetales, such as *Treponema* spp., and the like; and, protozoans, such as *Toxoplasma gondii*, and the like.

The biological sample will initially be pretreated in an aqueous phase, typically a buffer at a pH from about 5 to 9, in order to release both host and non-host nucleic acids, including both DNA and RNA. Conveniently, samples may be treated with an enzyme, usually a hydrolase, such as lysozyme, lyticase, or mutanolysin, typically at concentrations in the range from about 1 to 50 mg/ml, or with proteolytic enzymes, e.g., proteases such as proteinase K, pronase, pepsin or papain, typically at concentrations in the range from about 10 µg/ml to 20 mg/ml, more usually from about 0.1 mg/ml to 2 mg/ml, in order to disrupt the microorganism coat or envelope. Conveniently, microorganism extracts containing hydrolases, proteases, and/or cellulases may be employed. Exemplary microorganism extracts include those from *Aspergillus niger Cytophagia Rhizoctonia solani, Frichoderma harzianium*, and the like.

Samples will be separately or simultaneously treated with a mild detergent to denature or dissociate nucleic acid-protein complexes in order to release the nucleic acids. Suitable detergents include both anionic, cationic, and non-ionic detergents, such as sodium dodecyl sulfate (SDS) and cetyltrimethylammonium bromide (CTAB), and Triton X-100, at concentrations from about 0.05 to 2% by weight, more usually 0.05 to 0.15% by weight. The pretreated sample will be left to incubate for a period of from about 15 minutes to 1 hour at a temperature in the range from about 20° C to 50° C.

The pretreatment just described results in solubilization of a relatively large amount of biological material in addition to the desired nucleic acids. It is an object of the present invention to separate the nucleic acids from this complex mixture with a maximum recovery of the nucleic acid materials and a minimum carry-over of the potentially contaminating materials.

Optionally, a pre-extraction step may be performed with an anionic or non-ionic detergent in an organic solvent to separate proteins and cationic contaminants in particularly complex samples. Suitable anionic detergents include SDS, lauryl sulfates, lauryl phosphates, caprylate salts, cholate salts, 1-decanesufonate salts, deoxycholate salts, glycocholate salts, glycodeoxychloate salts, taurocholate salts, taurodeoxycholate salts, and the like. Preferred is the use of SDS at from about 1 to 5% in organic solvents, such as butanol or hexanol/octane (1:9).

Extraction of the nucleic acids is then performed by adding one or more cationic detergents, typically quaternary ammonium salts, quaternary phosphonium salts, amine oxides, pyridinium-derivatives, and the like, to the aqueous pretreatment or pre-extraction medium. The cationic detergent is usually dissolved in an organic phase, typically an alcohol or alcohol/alkane as described in more detail hereinbelow. The organic phase including the cationic detergent and the aqueous pretreatment medium are mixed so that the cationic detergent is able to form complexes with the anionic nucleic acids. Such complexes are sparingly soluble in low salt aqueous solutions and will partition into the organic phase once the mixture is allowed to equilibrate and separate into aqueous and organic phases.

Optionally, the cationic detergent may be added to the aqueous pretreatment medium prior to combination with the organic phase. The final cationic detergent concentration in the medium will typically

be in the range from about 0.1 to 5% by weight, resulting in the precipitation of nucleic acid-detergent complexes (which are substantially insoluble in low salt aqueous media). The cationic detergent added will usually, although not necessarily, be dissolved in water prior to addition to the pretreatment medium. After addition of the cationic detergent, the insoluble complexes may be separated from the aqueous pretreatment medium, typically by centrifugation or filtration. The separated complexes may then be combined with the organic phase where the complexes will dissolve. The nucleic acids may then be separated from the organic phase as described in more detail hereinbelow.

Usually, a chelant capable of complexing with bivalent or multivalent endogenous cations will be present in the extraction medium. The chelant will inhibit binding between the endogenous cations and polyanionic nucleic acids. Such binding by endogenous cations blocks binding by the cationic detergent and inhibits subsequent extraction of the nucleic acids. Suitable chelants include ethylenediaminetetraacetic acid (EDTA) ethylene glycol bis ($\beta$-aminoethyl ether) tetraacetic acid (EGTA), 8-hydroxy-2-methylquinoline, 8-hydroxyquinoline-5-sulfonic acid, 1,10-phenanthroline, 8-quinolinol, salicylate, and the like, usually being present in the extraction medium at from about 0.005 to 0.3M, more usually being present at from about 0.01 to 0.05M.

Suitable cationic detergents include those having cations which are soluble in an organic solvent and substantially less soluble in water. The detergent cations are capable of binding the polyanionic nucleic acids to form a complex which will preferentially migrate into the organic phase as a complex in an organic-aqueous phase mixture. Suitable detergents include quaternary ammonium salts, quaternary phosphonium salts, amine oxides, pyridinium-derivatives, and the like. Primary, secondary, and tertiary amines may also be suitable as detergents when protonated.

Quaternary ammonium and phosphonium salts will generally have the following formula:

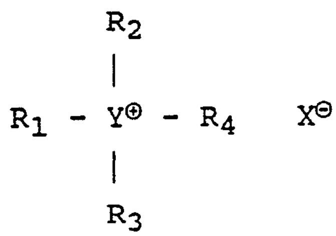

wherein Y is N or P, and R is aliphatic, alicyclic, or aromatic usually having at least about 6 carbons, more usually being an alkyl or alkenyl group having at least about 14 carbons, and frequently being 16 carbons or more. $R_2$, $R_3$, and $R_4$ are hydrogen, aliphatic, alicyclic, alkoxy or aromatic, usually being alkyl, alkenyl, or aryl, having 9 or fewer carbons, more usually having 6 or fewer carbons, typically being methyl, ethyl, propyl, or phenyl. X is a halogen or other compatible anion, usually being bromide, chloride, or acetate.

Exemplary quaternary ammonium salts include cetyltrimethyl ammonium bromide, cetyltrimethyl ammonium chloride, cetyldimethylethylammonium bromide, benzyldimethylhexadecylammonium bromide, cetyldimethylethyl ammonium chloride, trimethyltallow ammonium chloride, trimethyltallow ammonium bromide, and the like. Exemplary phosphonium ammonium salts include tetraphenyl phosphonium bromide, tetrabutyl phosphonium chloride, and the like.

Amine oxides will generally have the formula:

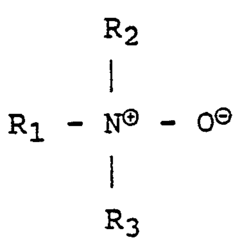

wherein $R_1$ is aliphatic, alicyclic, or aromatic, having at least about 6 carbons, usually having at least about 10 carbons, and preferably having in the range from about 12 to 18 carbons. $R_2$ and $R_3$ are hydrogen, aliphatic, alicyclic, alkoxy or aromatic, usually being alkyl or alkoxy having fewer than 9 carbons, more usually having fewer than 6 carbons.

Exemplary amine oxides include bis(2-hydroxyethyl) cocoamine oxide, bis(2-hydroxyethyl) tallowamine oxide, and the like.

Pyridinium-type cationic detergents will generally have the following formula:

$N^{\oplus}$ - R $X^{\ominus}$

wherein R is aliphatic, alicyclic, or aromatic, having at least about 6 carbons, usually having at least about 10 carbons, and preferably having from about 12 to 18 carbons.

Exemplary pyridinium-type cationic detergents include cetylpyridinium chloride, cetylpyridinium bromide, laurylpyridinium chloride, and the like.

Solvents suitable as the organic phase in the method of the present invention include organic solvents capable of solvating the nucleic acid complex formed as described above. The solvent will usually be a polar organic solvent, more usually being an alcohol or alcohol/alkane mixture. Suitable alcohols include monohydric aliphatic alcohols having the formula $CH_3$ $(CH_2)_nOH$, where n = 1 to 20, including methanol, ethanol, propanol, butanol, pentanol, hexanol, heptanol, octanol, isomers thereof, and the like. Particularly suitable is 1-butanol. Suitable alcohol/alkane mixtures include the above listed alcohols combined with an alkane having the formula $CH_3(CH_2)_n$ $CH_3$, where n = 5 to 20, including pentane, hexane, heptane, octane, nonane, isomers thereof, and the like. The weight ratio of alcohol:alkane will usually be in the range from about 1:5 to 1:20. Particularly suitable is a heptanol/octane (1:9) mixture.

In performing the method of the present invention, it is necessary that an aqueous phase containing the biological specimen be combined with the organic phase and a preselected concentration of the detergent in order to effect the initial separation of the nucleic acids into the organic phase. The concentration of the detergent in the organic phase will usually be in the range from about 0.001 to 0.5M, typically being about 0.01 to 0.1M. The relative amounts of the aqueous phase and the organic phase are not especially critical, usually being in the range from about 1:3 to 3:1 aqueous phase:organic phase (by volume), more usually being in about equal proportions by volume. The cationic detergent salt may be dissolved in the organic phase initially, or may be added to the combined aqueous and organic phases. The combined aqueous and organic phases will then be mixed, typically by shaking, to contact the detergent cations with the polyanionic nucleic acids. After mixing, typically for from about 30 seconds to 5 minutes, the resulting suspension is allowed to separate, typically for a period of from about 10 minutes to 2 hours. Optionally, the separation step may be assisted by centrifugation to reduce the time required, usually requiring about 2 to 30 minutes of from 200 to 5000x g.

Once the separation is achieved, the organic phase containing the nucleic acid complexes may be removed, leaving the undesirable biological material from the sample in the aqueous phase.

The nucleic acids are then returned to a second aqueous phase by separating the organic phase, typically by decanting, and mixing with the second aqueous phase. A preselected concentration of an inorganic salt, such as sodium chloride, potassium chloride, calcium chloride, magnesium chloride, sodium acetate, ammonium chloride, ammonium acetate, ammonium sulfate, or the like, is present in the second aqueous phase or may be added thereto. The salt concentration will usually be in the range from about 0.1 to 1 M, more usually being in the range from about 0.4 to 0.6M. The volume of the second aqueous phase added is not critical, typically being in the range from about 1:6 to 2:1 aqueous phase:organic phase (by volume), more usually being about 1:4. After allowing the phases to separate, the organic phase can be removed, and the aqueous phase can be used for nucleic acid hybridization testing in a conventional manner.

In some cases, it may be desirable to repeat the separation process just described by combining the second aqueous phase with an additional organic phase in the presence of the same or a different quaternary ammonium or phosphonium salt. The resulting nucleic acid-quaternary ammonium complexes are partitioned into the additional organic phase and may be reintroduced into a third aqueous phase in the manner just described. The process may be repeated as many times as necessary to achieve a desired level of purity of the nucleic acids.

Conventional techniques for performing nucleic acid hybridization testing for the detection of pathogenic microorganisms are well known in the art and described in generally circulated treatises, such as Hames and Higgens, Nucleic Acid Hybridization: A Practical Approach, IRL Press (1985).

An extraction device 10 for performing separation of nucleic acids according to the present invention without centrifugation is illustrated in Fig. 1. The device 10 includes a pair of vertical tubes 12 and 14 joined by a generally horizontal connector 16. The interior surfaces of the device 10 are siliconized by coating with a silane dissolved in an organic solvent followed by conventional evaporation techniques. A pump 18 is connected to circulate fluid from the upper portion of tube 12 to the lower portions of tubes 12 and 14. A stir bar 20 is provided at the bottom of each tube 12 and 14, and the upper halves of the tubes are interconnected by the horizontal connector 16. The dimensions of the device 10 are not critical, with a tube

diameter in the range from about 0.5 to 2 cm and a height in the range from about 2 to 10 cm generally being suitable. The device 10 is employed as follows.

One to ten milliliter of a clarified aqueous lysate (prepared from a biological samples as described above) is placed in the left tube 12 of the extraction device 10. A high salt aqueous phase of 1 to 10 ml is placed in the right tube 14, while 2 to 20 ml of organic solvent containing 0.001 to 0.5M of the cationic detergent is then overlaid so that the return tube 22 is completely filled with the organic phase. The organic phase is pumped from the upper end of tube 12 at a rate of 0.1 to 10 ml per minute simultaneously to the lower levels of both aqueous phases, at identical or different rates, for 10 to 30 minutes. Nucleic acids are thus transferred from the aqueous phase in tube 12 to the high salt solution in tube 14, with equilibrium being maintained by the return tube 22. After the extraction is complete, the high salt solution may be separated and employed for hybridization testing.

Alternatively, separate siliconized test tubes may be employed for continuous separation as illustrated in Fig. 2 and described as follows.

One to ten milliliters of the clarified aqueous lysate is placed in a first test tube 30 of appropriate size. On top of the aqueous phase is placed 1 to 3 volumes, usually 1 volume, of an organic solvent containing 0.001 to 0.5M of a suitable cationic detergent, usually about 0.3M. The top organic phase is circulated by a pump 32 at a rate of 0.1 to 20 ml per minute to the lower level of the aqueous phase for 5 to 30 minutes while the nucleic acids are transferred from the aqueous phase to the organic phase (Fig. 2A). The organic phase is then pumped to a second test tube 34 (Fig. 2B) containing 0.25 to 3 volumes of a high salt concentration aqueous medium, typically from 0.3 to 1 M, usually at about 0.4 M salt. The top organic phase is pumped at a rate of 0.1 to 20 ml per minute for 5 to 30 minutes to the lower level of the aqueous phase while the nucleic acids are transferred back to the aqueous phase. The aqueous phase may then be recovered for hybridization testing.

The following examples are offered by way of illustration, not by way of limitation.

## EXPERIMENTAL

### Example 1

A 2 gram fecal sample was uniformly suspended with 50 mM glucose, 0.2 M EDTA (pH 7.49), and 25 mM Tris-HCl (pH 8.0). The solution was then adjusted to 20 mg/ml lysozyme and incubated at 23° C for 15 minutes. Sodium dodecyl sulfate (SDS) was then added to a final concentration of 0.2% to lyse the suspended cells in the solution and pronase was then added to give a concentration of 1 mg/ml. The suspension was incubated a further 60 minutes at 23° C, at which time insoluble material was removed by centrifugation at 1200 x g for 5 minutes. The aqueous supernatent was carefully decanted and the pellet discarded.

The aqueous phase of the fecal extract was then mixed intimately by vigorous vortexing for 30 seconds with an equal volume of hexanol-octane (1:9) containing 3% cetyltrimethylammonium bromide (CTAB) (w/v), followed by centrifugation at 2000 x g for 2 minutes. DNA recovery of 96% was observed in the organic phase.

### Example 2

Same as Example 1 except that 0.1% CTAB was used instead of 0.2% SDS. DNA recovery of 70% was observed in the organic phase.

### Example 3

Same as Example 1 except that 0.1% CTAB was used instead of 0.2% SDS, and, prior to the organic extraction, CTAB was added to the aqueous phase to bring the final concentration to that observed in the organic phase. DNA recovery of 80% was observed in the organic phase.

Example 4

To feces (100 mg) were added 1 ml lysing buffer (50 mM glucose, 10 mM EDTA pH 8.0, 25 mM Tris-HCl pH 8.0), 0.8 ml of 0.5 M EDTA, 0.2 ml $H_2O$, and 20 mg lysozyme. The mixture was incubated at room temperature for 15 minutes, and then was adjusted to 0.2% SDS and 1 mg/ml proteinase K concentration. After incubation for 1 hour at room temperature, it was centrifuged at 2000 x g in an Eppendorf centrifuge to clarify the solution. The supernatant was removed and 0.5 volume water-saturated butanol and 0.5 volume 10% SDS were added to the clarified solution. After mixing 50 times by inversion, the mixture was centrifuged (5 min., 2000 x g). The aqueous phase which contained 89% of the nucleic acid was extracted with 1 volume butanol and 1 volume water containing 8g CTAB (inversion 50x).

The phases were separated by centrifugation (5 min.; 2000 x g). The butanol phase contained 70% of the DNA which could be extracted into a 0.5 M sodium acetate solution in 55% yield.

Example 5

To the clarified solution prepared in Example 4 was added 1 volume water-saturated ethyl acetate. After mixing 50 times by inversion, the mixture was centrifuged (2000 x g; 5 min.). The aqueous phase, which contained 80% of the DNA, was extracted with 1 volume butanol and 1 volume water containing 5 g/100 ml CTAB (50 times inversion). The phases were separated by centrifugation ( 5 min.; 2000 x g). The butanol phase contained about 55% of the nucleic acid which could be extracted into a 0.4 M NaCl solution with a 45% recovery.

Example 6

The same as Example 5 except that the aqueous phase after the ethyl acetate extraction was mixed by 50 inversions with a hexanol:octane (1:9) containing 0.2 M CTAB. The phases were separated by centrifugation (5 min; 2000 x g). The organic phase contained about 80% of the nucleic acid, which could then be extracted into a 0.4 M NaCl solution with a 56% recovery.

Example 7

To the clarified solution prepared in Example 4 was added 2 volumes of 0.2 M CTAB in hexanol:octane (1:9). The organic phase contained about 89% of the nucleic acid after mixing by inversion (50x) and separation by centrifugation (5 min; 2000 x g). The nucleic acids were extracted from the organic phase by 1 volume 1M sodium acetate with a 60% yield.

DNA can be extracted from different biological samples with an unoptimized yield as shown in Table 1.

TABLE 1

| CTAB Extraction of Nucleic Acids from Biological Samples | | |
|---|---|---|
| Sample | Method | Unoptimized % Yield in Organic Phase |
| Blood | Example 4 | 40-50 |
| Urine | Example 4 | 45-60 |
| Urine | Example 7 | 60 |
| Serum | Example 4 | 45-70 |
| M16 RNA | Example 5 | 70 |
| 16S, 23S RNA (E. coli) | Example 5 | 65 |

Example 8

A 4 ml clarified aqueous lysate (CTAB was used as the lysing detergent) was placed in a 15 ml siliconized test tube and 1 volume of hexanol/octane (1:9) containing 3 g/100 ml CTAB was layered on the aqueous lysate. The top organic phase was pumped at a rate of 1 ml/min to the lower level of the aqueous phase for 10 min. when 80% of the nucleic acids were transferred to the organic phase. The organic phase was then layered over 1 volume of 0.4 M sodium acetate in a second test tube. The top organic phase was then pumped at a rate of 1 ml/min to the lower level of the aqueous phase for 15 minutes when 50% of the total nucleic acids were transferred to the aqueous salt solution. The example illustrates the method as described in reference to Fig. 2.

Although the foregoing invention has been described in some detail by way of illustration and example for purposes of clarity of understanding, it will be obvious that certain changes and modifications may be practiced within the scope of the appended claims.

## Claims

1. A method for extracting nucleic acids from a complex biological sample prior to performing a hybridization assay, said method comprising:
pretreating the sample to release nucleic acids and contaminating materials therefrom into a first aqueous phase;
combining the first aqueous phase with an organic phase in the presence of a preselected concentration of a cationic detergent, whereby the detergent forms complexes with nucleic acids which enter the organic phase leaving the contaminating materials in the first aqueous phase;
separating the organic phase including the nucleic acids; and
combining the separated organic phase with a second aqueous phase in the presence of a preselected concentration of an inorganic salt, whereby the complexes dissociate and the nucleic acids enter the second aqueous phase and are available for hybridization testing.

2. A method for extracting nucleic acids from a complex biological sample prior to performing a hybridization assay, said method comprising:
combining an organic phase produced by:

(a) pretreating the sample to release nucleic acids and contaminating materials therefrom into a first aqueous phase;

(b) combining the first aqueous phase with an organic liquid in the presence of a chelant and a preselected concentration of a cationic detergent selected from quaternary ammonium salts, the group consisting of phosphonium salts, amine oxides, and pyridinium-derivatives whereby the nucleic acids form complexes with the detergents which enter the resulting organic phase leaving the contaminating materials in the first aqueous phase; and

(c) separating the organic phase; with a second aqueous phase in the presence of a preselected concentration of an inorganic salt, whereby the nucleic acid-detergent complexes dissociate and the nucleic acids enter the second aqueous phase and are available for hybridization testing.

3. A method for extracting nucleic acids from a complex biological sample prior to performing a hybridization assay, said method comprising:
combining an extracted organic phase produced by:

(a) pretreating the sample to release nucleic acids and contaminating materials therefrom into a first aqueous phase;

(b) combining the first aqueous phase with an organic liquid in the presence of a chelant and a preselected concentration of an anionic detergent, whereby contaminating proteins form complexes with the detergent and pass into the organic liquid;

(c) separating the organic liquid from step (b);

(d) combining the aqueous phase from step (b) with an organic phase in the presence of a chelant and a preselected concentration of a cationic detergent, whereby the nucleic acids form complexes with the detergent and preferentially pass into the organic phase leaving contaminating materials in the aqueous phase;

(e) separating the aqueous phase to produce the extracted organic phase;

with a second aqueous phase in the presence of a preselected concentration of an inorganic salt, whereby the nucleic acid-detergent complexes dissociate and the nucleic acids enter the second aqueous phase and are available for hybridization testing.

4. A method for extracting nucleic acids from a complex biological sample prior to performing a hybridization assay, said method comprising:
pretreating the sample to release nucleic acids and contaminating materials therefrom into a first aqueous phase;
combining the first aqueous phase with a preselected concentration of a cationic detergent, whereby the detergent forms complexes with nucleic acids which precipitate leaving a substantial portion of the contaminating materials in the first aqueous phase;
separating the precipitated nucleic acid-detergent complexes from the first aqueous phase;
dissolving the precipitated nucleic acid-detergent complexes with an organic phase; and
combining the organic phase with a second aqueous phase in the presence of a preselected concentration of an inorganic salt, whereby the complexes dissociate and the nucleic acids enter the second aqueous phase and are available for hybridization testing.

5. A method as in claims 1, 2, 3, or 4, wherein the complex biological sample is selected from the group consisting of blood, plasma, serum, urine, sputum, lavage, saliva, semen, tissue and fecal samples.

6. A method as in claims 1, 2, 3, or 4, wherein the biological sample is suspected of containing microorganisms.

7. A method as in claim 6, wherein the biological sample is a fecal sample and the microorganism is selected from the group consisting of Adenovirus, Rotavirus, *Salmonella* spp., *Shigella* spp., *Campylobacter* spp., *Clostridium difficile, Escherichia coli, Yersinia enterolitica, Candida albicans, Giardia lamblia, Entamoeba histolytica*, and *Microsporidium* spp.

8. A method as in claim 5, wherein the biological sample is sputum or lavage and the microorganism is selected from the group consisting of Adenovirus, Respiratory Syncytial Virus, Human Papillomavirus, Human Immunodeficiency Virus, Human T-Cell Lymphotrophic Viruses, Cytomegalovirus, Hepatitis A and B Virus, Epstein-Barr Virus, *Streptococcus pyrogenes* (Group A beta-hemolytic streptococci), *Streptococcus pheumoniae, Haemophilus influenzae, Staphylococcus aureus, Klebsiella pneumoniae, Pseudomonas aeruginosa, Neisseria meningitidis, Neisseria gonorrhoea, Chlamydia trachomatis, Escherichia coli Mycobacterium tuberculosis, Spirochaetaies*, such as *Treponema* spp., *Candida albicans, Histoplasma capsulatum, Coccidiodes imitis*, and *Pneumocystis carinii.*

9. A method as in claim 6, wherein the biological sample is blood and the microorganism is selected from the group consisting of Human Immunodeficiency Virus, Hepatitus A and B Virus, Human T-Cell Lyumphotrophic Viruses, *Escherichia coli, Staphylococcus aureus, Klebsiella pneumoniae, Streptococcus pneumoniae, Pseudomonas aeruginosa Spriochaetales*, such as *Treponema* spp., and *Toxoplasma gondii.*

10. A method as in claims 1, 2, 3, or 4, wherein the sample is pretreated by addition of an enzyme in combination with a detergent.

11. A method as in claims 1, 2, 3, or 4, wherein the organic phase is an alcohol or alcohol/alkane mixture.

12. A method as in claim 10, wherein the alcohol has the formula $CH_3(CH_2)_nOH$, where $n = 1$ to 20, or is an isomer thereof.

13. A method as in claim 11, wherein the alcohol is combined with an alkane, having the formula $CH_3$-$(CH_2)_nCH_3$, where $n = 5$ to 20, or an isomer thereof.

14. A method as in claims 1, 2, 3, or 4, wherein the cationic detergent is a quaternary ammonium or phosphonium salt selected from the group consisting of cetyltrimethylammonium bromide, cetyltrimethyl ammoniumchloride, cetyldimethylethylammonium bromide, cetyldimethylethylammonium chloride, trimethyltallowammonium chloride, trimethyltallowammonium bromide, tetraphenylphosphonium bromide, and tetrabutylphosphonium chloride.

15. A method as in claims 1, 2, 3, or 4, wherein the cationic detergent is an amine oxide selected from the group consisting of bis(2-hydroxyethyl) cocoamine oxide and bis(2-hydroxyethyl) tallowamine oxide.

16. A method as in claims 1, 2, 3, or 4, wherein the cationic detergent is a pyridinium-derivative selected from the group consisting of cetylpyridinium bromide, cetylpyridinium chloride, and laurylpyridinium chloride.

17. A method as in claims 1, 2, 3, or 4, wherein the cationic detergent is present in the aqueous phase at a concentration in the range from about 0.001M to 0.5M.

18. A method as in claims 1, 2, 3, or 4, wherein the inorganic salt is selected from the group consisting of sodium chloride, potassium chloride, magnesium chloride, calcium chloride, sodium acetate, ammonium chloride, ammonium acetate, and ammonium sulfate.

19. A method as in claim 17, wherein the inorganic salt is present in the second aqueous phase at a concentration in the range from about 0.3M to 1M.

20. A method as in claims 1, 2, 3, or 4, wherein the sample is extracted with a chelant in an organic solvent prior to pretreatment to release the nucleic acids and residue materials.

21. A method as in claims 2, 3, or 4, wherein the chelant is selected from the group consisting of EDTA, EGTA, 8-hydroxy-2-methylquinoline, 8-hydroxyquinoline-5-sulfonic acid, 1,10-phenanthroline, 8-quinolinol, and salic acid and is present at from about 0.005M to 0.3M.

22. A method as in claims 1, 2, 3, or 4, further comprising separating the organic phase from the second aqueous phase by centrifugation.

23. A method as in claim 4, wherein the precipitated nucleic acid-detergent complexes are separated from the first aqueous phase by filtration or centrifugation prior to dissolving.

24. A method for performing nucleic acid hybridization assays to detect the presence of microorganisms in complex biological samples, said method comprising:
pretreating the sample to release nucleic acids and contaminating materials from the microorganisms into a first aqueous phase;
combining the first aqueous phase with an organic phase in the presence of a preselected concentration of a cationic detergent, whereby complexes of the nucleic acids form and enter the organic phase leaving the contaminating materials substantially in the first aqueous phase;
separating the organic phase;
combining the separated organic phase with a second aqueous phase in the presence of a preselected concentration of an inorganic salt, whereby the complexes dissociate and the nucleic acids enter the second aqueous phase;
combining at least one detectable nucleic acid probe with the second aqueous phase under hybridization conditions; and
detecting the presence or absence of probe hybridization complexes.

25. A method as in claim 24, wherein the complex biological sample is selected from the group consisting of blood, plasma, serum, urine, sputum, lavage, saliva, semen, tissue, and fecal samples.

26. A method as in claim 24, wherein the biological sample is a fecal sample and the microorganism is selected from the group consisting of Adenovirus, Rotavirus, *Salmonella* spp., *Shigella* spp., *Campylobacter* spp., *Clostridium difficile, Escherichia coli, Yersinia enterolitica, Candida albicans, Giardia lamblia, Entamoeba histolytica*, and *Microsporidium* spp.

27. A method as in claim 23, wherein the biological sample is sputum or lavage and the microorganism is selected from the group consisting of Adenovirus, Respiratory Syncytial Virus, Human Papillomavirus, Human Immunodeficiency Virus, Human T-Cell Lymphotrophic Viruses, Cytomegalovirus, Hepatitis A and B Virus, Epstein-Barr Virus, *Streptococcus pyrogenes* (Group A beta-hemolytic streptococci), *Streptococcus pheumoniae, Haemophilus influenzae, Staphylococcus aureus Klebsiella pneumoniae Pseudomonas aeruginosa, Neisseria meningitidis, Neisseria gonorrgoea, Chlamydia trachomatis, Escherichia coli, Mycobacterium tuberculosis, Spirochaetales*, such as *Treponema* spp., *Candida albicans, Histoplasma capsulatum Coccidiodes imitis*, and *Pneumocystis carinii.*

28. A method as in claim 24, wherein the biological sample is blood and the microorganism is selected from the group consisting of Human Immunodeficiency Virus, Hepatitus A and B Virus, Human T-Cell Lyumphotrophic Viruses, *Escherichia coli, Staphylococcus aureus, Klebsiella pneumoniae, Streptococcus pneumoniae, Pseudomonas aeruginosa, Spriochaetales*, such as *Treponema* spp., and *Toxoplasma gondii.*

29. A method as in claim 24, wherein the sample is pretreated by addition of an enzyme in combination with a detergent.

30. A method as in claim 24, wherein the organic phase is an alcohol or alcohol/alkane mixture.

31. A method as in claim 30, wherein the alcohol has the formula $CH_3(CH_2)_nOH$, where $n = 1$ to 20, or is an isomer thereof.

32. A method as in claim 30, wherein the alkane has the formula $CH_3(CH_2)_nCH_3$, where $n = 5$ to 20, or is an isomer thereof.

33. A method as in claim 24, wherein the cationic detergent is a quaternary ammonium or phosphonium salt selected from the group consisting of cetyltrimethyl ammonium bromide, cetyltrimethyl ammonium chloride, cetyldimethylethyl ammonium bromide, cetyldimethylethyl ammonium chloride, cetylpyridinium bromide, cetylpyridinium chloride, trimethyltallow ammonium chloride, trimethyltallow ammonium bromide, and the like. Exemplary phosphonium ammonium salts include tetraphenyl phosphonium bromide, tetraphenyl phosphonium chloride, and the like.

34. A method as in claim 24, wherein the cationic detergent is an amine oxide selected from the group consisting of bis(2-hydroxyethyl) cocoamine oxide and bis(2-hydroxyethyl) tallowamine oxide.

35. A method as in claim 24, wherein the cationic detergent is a pyridinium-derivative selected from the group consisting of cetylpyridinium bromide, cetylpyridinium chloride, and laurylpyridinium chloride.

36. A method as in claim 24, wherein the cationic detergent is present in the first aqueous phase at a concentration in the range from about 0.001M to 0.5M.

37. A method as in claim 24, wherein the inorganic salt is selected from the group consisting of sodium chloride, potassium chloride, magnesium chloride, calcium chloride, sodium acetate, ammonium chloride, ammonium acetate, and ammonium sulfate.

38. A method as in claim 37, wherein the inorganic salt is present in the second aqueous phase at a concentration in the range from about 0.3M to 1M.

39. A method as in claim 24, wherein the sample is extracted with an anionic detergent in an organic solvent prior to pretreatment to release the nucleic acids and residue materials.

40. A method as in claim 24, wherein the first aqueous phase is further combined with a preselected concentration of a chelant.

41. A method as in claim 40, wherein the chelant is selected from the group consisting of EDTA, EGTA, 8-hydroxy-2-methylquinoline, 8-hydroxyquinoline-5-sulfonic acid, 1,10-phenanthroline, 8-quinolinol, and salicylate and is present at a concentration in the range from about 0.005M to 0.3M.

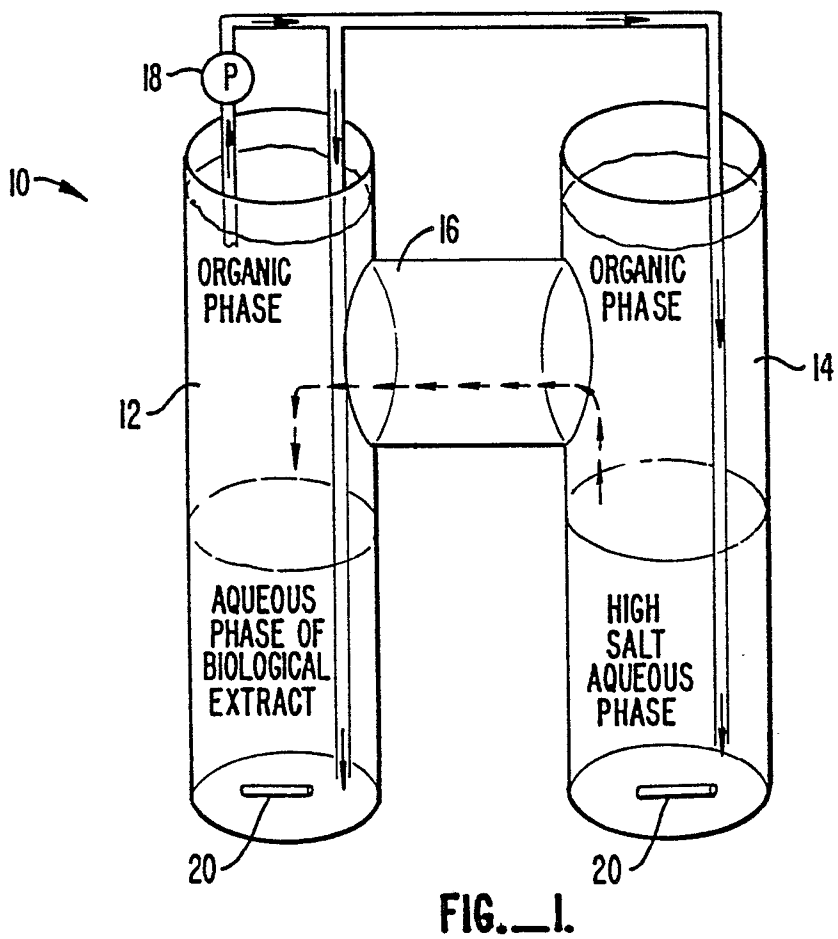


FIG._1.

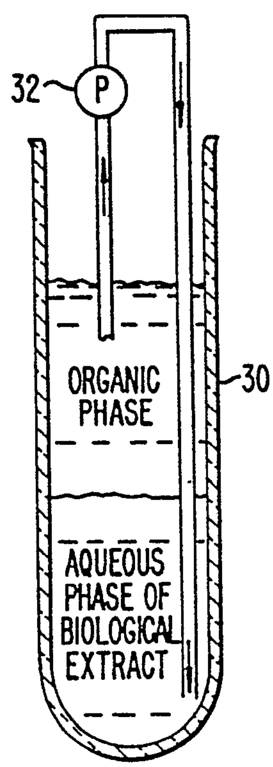


FIG._2A.

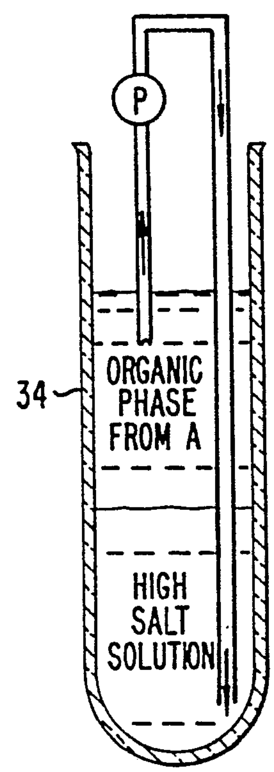


FIG._2B.